# EUROPÄISCHE PATENTSCHRIFT

(11) **EP 1 032 315 B1**
(45) Veröffentlichungstag und Bekanntmachung des Hinweises auf die Patenterteilung: **02.01.2004**
(21) Anmeldenummer: 98958871.0
(22) Anmeldetag: 24.10.1998
(51) Int. Cl.: A61B 17/128

(54) **MAGAZIN FÜR EIN CHIRURGISCHES CLIPANLEGEGERÄT**
MAGAZINE FOR A SURGICAL CLIPPING DEVICE
MAGASIN POUR DISPOSITIF DE POSE D'AGRAFES

(30) Priorität: 26.11.1997 DE 19752331
(43) Veröffentlichungstag der Anmeldung: 06.09.2000
(73) Patentinhaber: Aesculap AG & Co. KG, 78532 Tuttlingen (DE)
(72) Erfinder: KIENZLE, Karl-Ernst, D-78194 Immendingen (DE); MAYENBERGER, Rupert, D-78239 Rielasingen (DE); WEISSHAUPT, Dieter, D-78194 Immendingen (DE)
(74) Vertreter: Böhme, Ulrich, Dr. Dipl.-Phys.
(86) Internationale Anmeldenummer: PCT/EP1998/006775
(87) Internationale Veröffentlichungsnummer: WO 1999/027859

(56) Entgegenhaltungen:
- DE-C- 3 704 760
- US-A- 4 854 317

## Beschreibung

Die Erfindung betrifft ein Magazin für ein chirurgisches Clipanlegegerät mit einem Aufnahmeraum für hintereinander angeordnete und im Aufnahmeraum zu dessen offenem Ende hin verschiebbare Clips, die zwei über einen Steg verbundene, gegen eine elastische Kraft in eine Öffnungsstellung auseinanderschwenkbare Arme und diesen zugeordnete, über den Steg nach hinten vorstehende Vorsprünge aufweisen, und mit zwei einander gegenüberliegenden, zangenartig gegeneinander bewegbaren, an den Vorsprüngen des jeweils vordersten Clips anlegbaren Öffnungswerkzeugen.

Derartige Magazine werden verwendet, um aus ihnen eine größere Anzahl von Clips zu applizieren, beispielsweise zum Abklemmen der Kopfhaut bei Kopfoperationen. Für diesen Zweck sind bereits Clipanlegegeräte mit Magazinen bekannt, in denen die Clips hintereinanderliegend aufbewahrt werden und aus denen die Clips schrittweise nach vorne vorgeschoben werden. Der jeweils vorderste Clip wird von zangenartigen Öffnungswerkzeugen erfaßt, die an derartigen Clipanlegegeräten beweglich und ortsfest gelagert sind, und diese zangenartigen Öffnungswerkzeuge öffnen den Clip und legen ihn an der gewünschten Stelle an (DE-PS 37 04 760). Bei derartigen Geräten sind die Öffnungswerkzeuge Teil der Geräte, auswechselbar sind lediglich die Magazine mit den darin aufgenommenen Clips.

Es ist Aufgabe der Erfindung, ein gattungsgemäßes Magazin so auszugestalten, daß der Aufbau derartiger Clipanlegegeräte insgesamt vereinfacht werden kann, insbesondere dadurch, daß die Clipanlegegeräte keine eigene Mechanik zum Öffnen und Anlegen der Clips benötigen.

Diese Aufgabe wird bei einem Magazin der eingangs beschriebenen Art erfindungsgemäß dadurch gelöst, daß die Öffnungswerkzeuge scharnierartig mit einem im Magazin parallel zur Verschieberichtung der Clips verschiebbaren Vorschubelement verbunden sind und bei dessen in Richtung auf das offene Ende des Aufnahmeraumes gerichteter Vorschubbewegung an einer Umlenkfläche des Magazins zur Anlage gelangen und dadurch quer zur Verschieberichtung zum jeweils gegenüberliegenden Öffnungswerkzeug eingeschwenkt werden.

Bei einer solchen Lösung sind also die als Scharnierelemente ausgebildeten Öffnungswerkzeuge Teil des Magazins und sie werden in einfacher Weise dadurch gelagert, daß sie scharnierartig mit einem in Längsrichtung des Magazins verschiebbaren Vorschubelement verbunden werden. Zum Einschwenken dieser Scharnierelemente wird die Vorschubbewegung der Vorschubelemente ausgenutzt, und diese Vorschubbewegung wird durch die Anlage der scharnierartig mit den Vorschubelementen verbundenen Öffnungswerkzeuge an einer Umlenkfläche des Magazins erzeugt. Dadurch ergibt sich ein besonders einfacher Aufbau sowohl des Magazins als auch des gesamten Clipanlegegerätes, denn es genügt, am Clipanlegegerät einen Mechanismus vorzusehen, der die Vorschubelemente im Magazin vor- und wieder zurückschiebt. Spezielle Öffnungswerkzeuge am Clipanlegegerät selbst sind nicht mehr notwendig.

Durch diese Konstruktion können auch sehr hohe Öffnungskräfte erzeugt werden, da das Scharnierelement und das Vorschubelement nach Art eines Kniegelenkes zusammenwirken. Die Vorschubbewegung wird insbesondere am Ende der Vorschubbewegung in eine sehr geringe Winkelverschwenkung übersetzt, und diese sehr geringe Winkelverschwenkung führt zu einer geringen Verschiebung des Öffnungswerkzeuges an dem zu öffnenden Clip, d. h. es tritt eine Untersetzung des Verschiebeweges auf und damit eine Erhöhung der entsprechenden Schiebekraft. Die nach innen gerichteten Öffnungskräfte werden im übrigen im wesentlichen von der Führung des Vorschubelementes übernommen, an die sich das Vorschubelement anlegt und an dem sich das Vorschubelement damit abstützt.

Besonders vorteilhaft ist es, wenn die Öffnungswerkzeuge gegen eine elastische Rückstellkraft nach außen verschwenkbar sind, die geringer ist als die zum Öffnen der Clips nötige Kraft. Durch diese Rückstellkraft werden also die Öffnungswerkzeuge nach innen gegen die Clips verschwenkt und legen sich mit ihren freien Kanten an die Clips an, insbesondere an die Vorsprünge des jeweils vordersten Clips, sie öffnen jedoch den Clip noch nicht, da diese elastische Rückstellkraft kleiner ist als die notwendige Öffnungskraft. Diese elastische Anlage der Öffnungswerkzeuge am vordersten Clip führt dazu, daß der vorderste Clip zwischen den Öffnungswerkzeugen gehalten wird, bis er nach vorne in die Applizierposition vorgeschoben wird.

Eine besonders vorteilhafte Ausgestaltung ergibt sich, wenn die Öffnungswerkzeuge über einen geschwächten Scharnierbereich einstückig mit den Vorschubelementen verbunden sind.

Insbesondere können die Öffnungswerkzeuge und das Vorschubelement dann aus einem elastischen Kunststoffmaterial bestehen, so daß sich eine billige Konstruktion des Magazins ergibt, das in diesem Falle auch als Wegwerfteil konzipiert werden kann.

Gemäß einer bevorzugten Ausführungsform ist vorgesehen, daß die Umlenkfläche durch die sich zu beiden Seiten an das offene Ende des Aufnahmeraumes anschließende Stirnwand des Magazins gebildet wird.

Eine ganz besonders vorteilhafte Konstruktion ergibt sich, wenn das Vorschubelement an die Clips anlegbare Mitnehmer zum schrittweisen Vorschieben der Clips aufweisen.

Damit übernimmt das Vorschubelement eine zusätzliche Funktion, nämlich die des Vorschubs aller im Magazin gelagerten Clips, die bei jeder Vorschubbewegung des Vorschubelementes um eine Cliplänge vorgeschoben werden, wobei gleichzeitig diese Vorschubbewegung des Vorschubelementes auch das Schließen der Öffnungswerkzeuge hervorruft.

Bei einer bevorzugten Ausführungsform ist vorgesehen, daß sich die Öffnungswerkzeuge beim Vorschieben des Vorschubelementes an den Vorsprüngen des vordersten Clips anlegen, bevor die Mitnehmer des Vorschubelementes sich an folgende Clips anlegen. Dadurch wird erreicht, daß der jeweils vorderste Clip weiter vorgeschoben wird als die übrigen Clips und damit einen Abstand vom nachfolgenden Clip aufweist, der ausreichend ist für das Einschieben der Öffnungswerkzeuge.

Die Mitnehmer sind aus dem Aufnahmeraum ausrückbar ausgebildet, so daß beim Zurückschieben der Vorschubelemente die beim Vorschieben des Vorschubelementes vorgeschobenen Clips nicht wieder zurückgezogen werden. Die Mitnehmer können dabei in unterschiedlicher Weise aus dem Aufnahmeraum ausgerückt werden, bei einer bevorzugten Ausführungsform ist vorgesehen, daß die Mitnehmer beim Zurückziehen des Vorschubelementes elastisch an den Clips entlanggleiten, d. h. die Clips selbst drükken in diesem Falle die Mitnehmer elastisch nach außen.

Insbesondere können die Mitnehmer als Federzungen ausgebildet sein, die in Vorschubrichtung schräg in den Aufnahmeraum hineinragen. Neben der Mitnahmefunktion kommt den Federzungen bei dieser Ausgestaltung auch noch die Aufgabe zu, die Clips längs ihres Verschiebeweges zu führen und gegen eine Verkipppung zu sichern.

Bei einer bevorzugten Ausführungsform ist vorgesehen, daß die Vorschubelemente als Halbschalen ausgebildet sind, die gemeinsam den kanalförmigen Aufnahmeraum für die Clips bilden.

Diese Halbschalen sind in Vorschubrichtung relativ zueinander festgelegt, beispielsweise durch formschlüssig zusammenwirkende Vor- und Rücksprünge, so daß bei der Verschiebung eines Vorschubelementes in Längsrichtung das andere Vorschubelement zwangsläufig mitgenommen wird.

Es ist dabei vorteilhaft, wenn die Halbschalen in einem länglichen Gehäuse des Magazins in dessen Längsrichtung verschiebbar gelagert sind.

Dabei kann vorgesehen sein, daß die Halbschalen durch das Gehäuse in gegenseitiger Anlage gehalten werden. Dadurch erhält man einerseits durch das die Halbschalen umgebende Gehäuse eine Führung der Halbschalen, andererseits ist es nicht notwendig, die beiden Halbschalen dauerhaft fest miteinander zu verbinden, es genügt die Anlage durch das umgebende Gehäuse, um die beiden Halbschalen die Clips umgebend festzulegen.

Die Clips können im Aufnahmeraum gegen ein ungewolltes Zurückschieben in verschiedener Weise gesichert werden, beispielsweise durch einen Reibschluß mit den Seitenwänden.

Gemäß einer besonders bevorzugten Ausführungsform ist vorgesehen, daß in den Aufnahmeraum aus diesem ausrückbare Rückhaltevorsprünge für die Clips hineinragen, die in Längsrichtung des Magazins festgelegt sind. Diese sind so angeordnet, daß die Clips bei ausgerückten Rückhaltevorsprüngen nach vorne verschoben werden können, bei eingerückten Rückhaltevorsprüngen aber an einer Rückbewegung gehindert sind.

Insbesondere können die Rückhaltevorsprünge federnd aus dem Aufnahmeraum herausbewegbar sein, d. h. die Clips drücken die Rückhaltevorsprünge beim Vorschieben federnd aus dem Aufnahmeraum heraus, schlagen aber bei einer Rückbewegung an den in den Aufnahmeraum eingefederten Rückhaltevorsprüngen an.

Vorteilhaft ist es, wenn die Rückhaltevorsprünge Federzungen sind, die in Vorschubrichtung schräg in den Aufnahmeraum hineinragen.

Bei einer bevorzugten Ausführungsform sind die Rückhaltevorsprünge an den Seitenwänden des Aufnahmeraumes angeordnet. Es ergibt sich dann eine Anordnung, bei der die Mitnehmer der Vorschubelemente beispielsweise auf der Oberseite und der Unterseite der Clips angeordnet sind, die Rückhaltevorsprünge dagegen an den beiden Seitenwänden.

Es ist vorteilhaft, wenn die Rückhaltevorsprünge an Trägern gehalten sind, die in den Aufnahmeraum seitlich eingeschoben und die axial unverschieblich am Magazin festgelegt sind. Dabei kann es sich beispielsweise um Metallstreifen handeln, die als Rückhaltevorsprünge federnd nach innen gebogene Ausklinkungen aufweisen.

Gemäß einer bevorzugten Ausführungsform ist vorgesehen, daß die Vorschubelemente an ihrer Außenseite in Längsrichtung aneinander anschließende Vertiefungen aufweisen, in die ein in Verschieberichtung der Clips vorund zurückschiebbarer Transportmitnehmer eingreift. Dieser Transportmitnehmer verschiebt die Vorschubelemente im Magazin reziprozierend hin und her, wobei einerseits die Clips jeweils um einen Schritt nach vorne geschoben werden, während andererseits der jeweils vorderste Clip zur Anlage geöffnet und wieder geschlossen wird.

Günstig ist es, wenn der Boden der Vertiefung an drei Seiten vom Vorschubelemente abgetrennt ist und den in den Aufnahmeraum hineinragenden Mitnehmer für die Clips bildet. Dadurch ergibt sich eine besonders einfache konstruktive Ausgestaltung für die Vorschubelemente.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht eines Clipanlegegerätes mit eingesetztem Magazin;

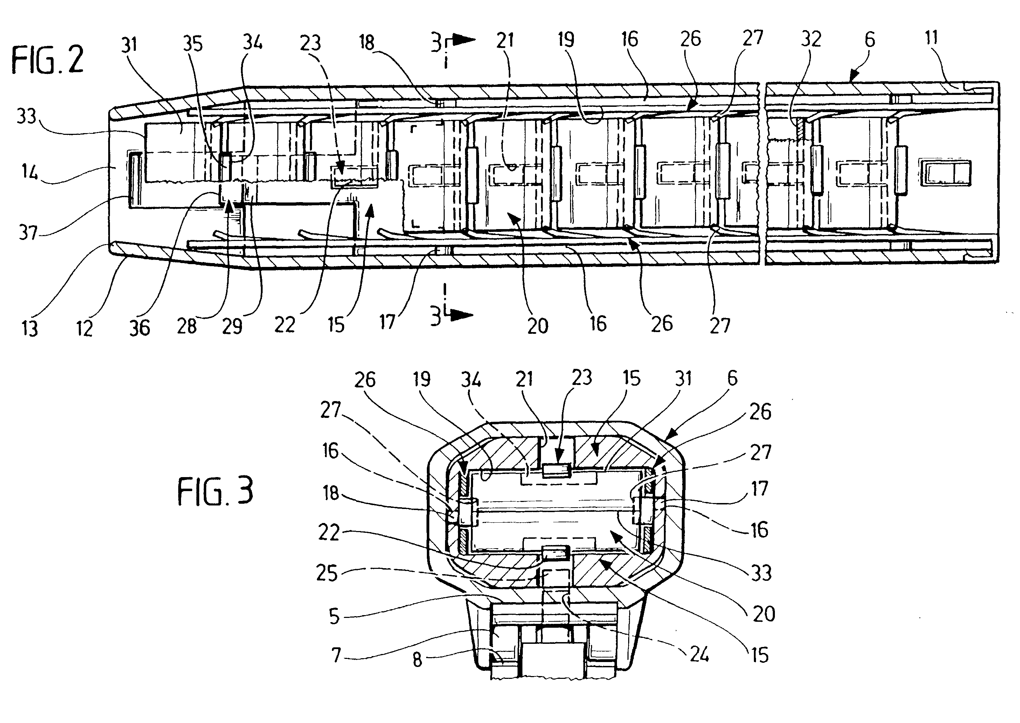

- Figur 2:: eine Schnittansicht längs Linie 2-2 in Figur 1;
- Figur 3:: eine Schnittansicht längs Linie 3-3 in Figur 2;
- Figur 4:: eine vergrößerte Teillängsschnittansicht des Magazins mit einem zum Applizieren vorgeschobenen Clip vor der Öffnung;
- Figur 5:: eine Ansicht ähnlich Figur 4 mit einem zum Applizieren vorgeschobenen und geöffneten Clip und
- Figur 6:: eine Ansicht ähnlich Figur 4 mit dem applizierten Clip und wieder zurückgezogenem Vorschubelement.

Das in der Zeichnung dargestellte Clipanlegegerät umfaßt ein Handstück 1 mit einem feststehenden Griffteil 2 und einem verschwenkbar daran gelagerten Betätigungsgriff 3, der durch eine am Griffteil 1 festgelegte Biegefeder 4 in eine vorderste Stellung verschwenkt wird.

Das Handstück 1 schließt zur Oberseite hin mit einer ebenen Auflagefläche 5 ab, auf der ein längliches Gehäuse 6 mit rechteckigem Querschnitt lösbar gehalten ist. Dieses Gehäuse 6 ist an der Auflagefläche 5 durch seitlich abstehende Querstifte 7, die in eine vorne offene Ausnehmung 8 des Handstückes 1 eingreifen, und durch eine Raste 9 gesichert, die in eine Rastausnehmung 10 des Griffteiles 2 eintaucht und durch geeignete, in der Zeichnung nicht dargestellte Betätigungsmittel gelöst werden kann.

Das Gehäuse 6 ist an seinem hinteren Ende 11 offen, an seinem vorderen Ende 12 wird es von einer Stirnwand 13 verschlossen, die eine sich über die gesamte Breite des Gehäuses erstreckende zentrale Austrittsöffnung 14 aufweist.

Im Innern des Gehäuses 6 sind zwei gleich aufgebaute, Halbschalen ausbildende Vorschubelemente 15 längsverschieblich gelagert. Diese Vorschubelemente 15 liegen mit ihren Seitenkanten 16 aneinander an und greifen dort mit Vorsprüngen 17 in Rücksprünge 18 des jeweils anderen Vorschubelementes 15 formschlüssig ein, so daß beide Vorschubelemente 15 dadurch in Längsrichtung des Gehäuses 6 relativ zueinander fixiert sind.

Die beiden mit ihrer offenen Seite einander zugewandten Vorschubelemente 15 füllen den Querschnitt des Gehäuses 6 vollständig aus und umgeben einen sich über ihre Länge erstreckenden Aufnahmeraum 19, der der Aufnahme von im Querschnitt U- oder C-förmigen Clips 20 dient.

Beide Vorschubelemente 15 weisen an ihrer Oberseite bzw. Unterseite eine Anzahl von Vertiefungen 21 auf, die im dargestellten Ausführungsbeispiel quaderförmig ausgebildet sind und längs der Mittellinie der Vorschubelemente 15 angeordnet sind. Der Boden 22 jeder Vertiefung 21 ist relativ dünn ausgebildet und an drei Seiten der Vertiefung 21 aufgetrennt, so daß der Boden 22 nur an der hinteren Querkanten mit dem Vorschubelement 15 verbunden ist. Außerdem ist der Boden 22 nach innen verformt, so daß diese Böden 22 schräg nach innen vorstehende, zungenförmige Mitnehmer 23 ausbilden, die in weiter unten näher erläuterter Weise an der Rückseite der Clips 20 zur Anlage kommen. Die Mitnehmer 23 sind federnd nach außen verschwenkbar, so daß sie dadurch aus dem Aufnahmeraum 19 nach außen ausrückbar sind.

In der Auflagefläche 5 des Handstückes 1 ist eine Durchbrechung 24 vorgesehen, durch die ein Transportmitnehmer 25 hindurchragt, der in eine der Vertiefungen 21 eines Vorschubelementes 15 eingreift. Beim Verschwenken des Betätigungsgriffes 3 wird dieser Transportmitnehmer 25 relativ zur Auflagefläche 5 nach vorne verschoben und nimmt dabei die beiden Vorschubelemente 15 mit. Es ist somit möglich, durch Verschwenken des Betätigungsgriffes 3 die Vorschubelemente nach vorne und durch Zurückschwenken des Betätigungsgrifes 3 die Vorschubelemente 15 wieder zurückzuschieben. Die Länge des Verschiebeweges ist dabei größer als die Länge eines Clips 20.

In den von den beiden halbschaligen Vorschubelementen 15 ausgebildeten Aufnahmeraum 19 sind längs der Seitenflächen des Aufnahmeraumes 19 streifen- oder plattenförmige Träger 26 eingeschoben, aus denen eine Reihe von Federzungen 27 herausgetrennt sind, die zum vorderen Ende des Gehäuses 6 hin schräg in den Aufnahmeraum 19 gerichtet sind. Die Träger 26 sind durch geeignete Mittel, z.B. Rastelemente, am Gehäuse 6 in Längsrichtung unverschieblich festgelegt, die Federzungen 27 bilden somit in den Aufnahmeraum 19 hineinragende, federnd aus diesem ausschwenkbare Rückhalteelemente. Der Abstand benachbarter Rückhalteelemente ist ebenso wie der Abstand benachbarter Mitnehmer an den Vorschubelementen 15 durch die Länge der Clips bestimmt und geringfügig größer als diese.

Am vorderen Ende ist an jedes der beiden Vorschubelemente 15 in der Verlängerung der Oberseite bzw. der Unterseite ein Wandabschnitt 28 angeformt, der mit dem übrigen Vorschubelement durch einen geschwächten Bereich 29 scharnierartig derart verbunden ist, daß der Wandabschnitt 28 um eine quer zur Längsrichtung des Aufnahmeraumes 19 verlaufende Schwenkachse nach innen verschwenkbar ist. Dieser Wandabschnitt 28 ist dabei in seiner Ruhestellung nach innen geschwenkt und kann gegen eine elastische Rückstellkraft nach außen verschwenkt werden.

Die in den Aufnahmeraum 19 hintereinander eingefüllten Clips 20 weisen zwei im wesentlichen parallel zueinander verlaufende Arme 31 auf, die über einen Steg 32 miteinander verbunden sind. Sie bestehen aus einem elastischen Material, beispielsweise einem sterilisierbaren Kunststoff, und durch die Eigenelastizität des Materials werden die beiden Arme 31 mit ihren freien Enden 33 gegeneinander gepreßt. Diese freien Enden 33 bilden Klemmleisten aus.

In der Verlängerung der beiden Arme 31 stehen nach hinten Vorsprünge 34 mit nach außen gerichteten Vertiefungen 35 über den Steg 32 über, diese Vorsprünge 34 dienen der Öffnung der Clips. Wenn diese Vorsprünge 34 einander angenähert werden, wird der bogenförmig in den Clip hineingebogene Steg 32 verformt und die Arme 31 öffnen sich gegen die elastische Rückstellkraft des Steges 32.

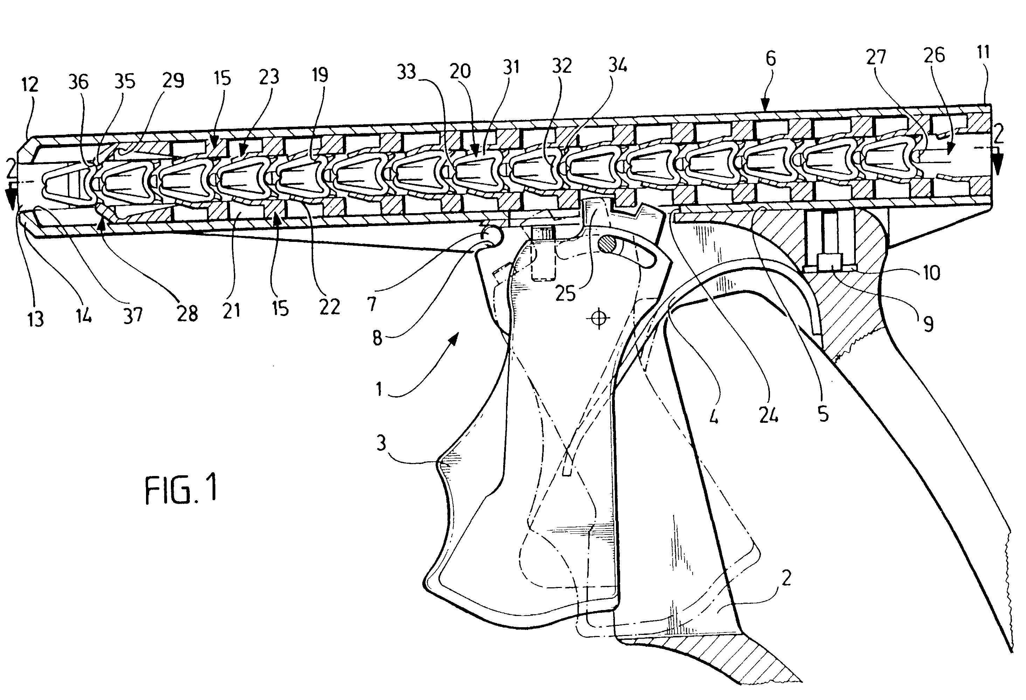

Beim Betrieb des beschriebenen Clipanlegegerätes wird ein das Gehäuse 6, die Vorschubelemente 15, die Träger 26 und eine Anzahl von in den Aufnahmeraum 19 hintereinander eingeschobenen Clips 20 aufnehmendes Magazin auf die Auflagefläche 5 des Handstückes 1 in der beschriebenen Weise aufgesetzt. Die beiden Vorschubelemente 15 befinden sich dabei in einer zurückgezogenen Stellung, die als federnde Rastzungen ausgebildeten Mitnehmer 23 liegen jeweils an den Armen 31 eines Clips 20 an und richten dadurch die Clips 20 relativ zum Aufnahmeraum aus (Figur 1).

Durch Betätigung des Betätigungsgriffes 3 werden die Vorschubelemente 15 nach vorne geschoben, und dabei legen sich die Mitnehmer 23 mit ihren freien Kanten an die Rückseiten des vor ihnen liegenden Clips 20 an, diese Clips 20 werden dadurch zusammen mit dem Vorschubelement 15 um eine Cliplänge nach vorne verschoben. Dabei bewegen sie die Federzungen 27 der Träger 26 elastisch nach außen und gleiten an diesen vorbei.

In der vordersten Position sind die Clips 20 dabei soweit vorgeschoben, daß die Federzungen 27 wieder in den Aufnahmeraum zurückfedern können und sich mit ihren freien Kanten unmittelbar hinter den Stegen 32 der vorbeigeschobenen Clips 20 positionieren.

Wenn nach dieser Vorschubbewegung der Vorschubelemente 15 diese nun zurückgezogen werden, werden die Clips 20 durch die eingefederten Federzungen 27 an einer Rückbewegung gehindert und in ihrer vorgeschobenen Position festgehalten. Die als Federzungen ausgebildeten Mitnehmer 23 gleiten bei der Rückbewegung der Vorschubelemente 15 auf den in dieser Weise gehaltenen Clips 20 auf und an diesen vorbei, bis sie an dem darauf folgenden Clip seitlich zur Anlage kommen und diesen in der eingangs beschriebenen Weise positionieren. Damit kann bei jeder Betätigung des Betätigungsgriffes 3 die Gesamtheit der Clips 20 um eine Cliplänge nach vorne in Richtung auf die Austrittsöffnung 14 des Aufnahmeraumes 19 verschoben werden.

Der jeweils vorderste Clip in der Reihe der im Aufnahmeraum 19 angeordneten Clips 20 wird nicht durch einen Mitnehmer 23 der Vorschubelemente 15 vorgeschoben, sondern durch die am vorderen Ende der Vorschubelemente 15 angeordneten Wandabschnitte 28.

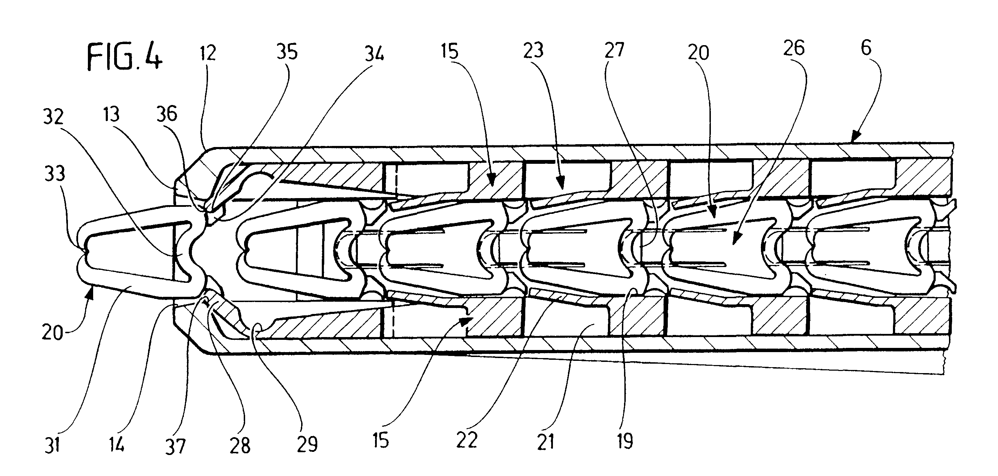

Diese greifen bei zurückgezogenen Vorschubelementen 15 mit ihren freien Kanten 36 in die Vertiefungen 35 der Vorsprünge 34 des Clips 20 ein und nehmen diesen Clip beim Vorschieben des Vorschubelementes 15 soweit mit, daß der Clip 20 mit seinen Armen 31 durch die Austrittsöffnung 14 aus dem Gehäuse 6 hervorsteht (Figur 4). Die Haltekraft wird dabei durch die Elastizität der nach innen gerichteten Wandabschnitte 28 aufgebracht, diese elastische Kraft ist ausreichend, um die Clips 20 zu halten, jedoch nicht ausreichend, die Clips 20 zu öffnen.

Beim weiteren Vorschieben der Vorschubelemente 15 legen sich die Wandabschnitte 28 an die die Austrittsöffnung 14 beiderseits begrenzenden Kanten 37 der Stirnwand 13 des Gehäuses 6 an und werden beim weiteren Vorschieben der Vorschubelemente 15 stärker nach innen verschwenkt, also mit ihren freien Kanten 36 einander angenähert. Die Kanten 37 der Stirnwand 13 bilden somit eine Umlenkfläche aus, die die Wandabschnitte 28 aufgrund der Vorschubbewegung der Vorschubelemente 15 nach innen verschwenkt.

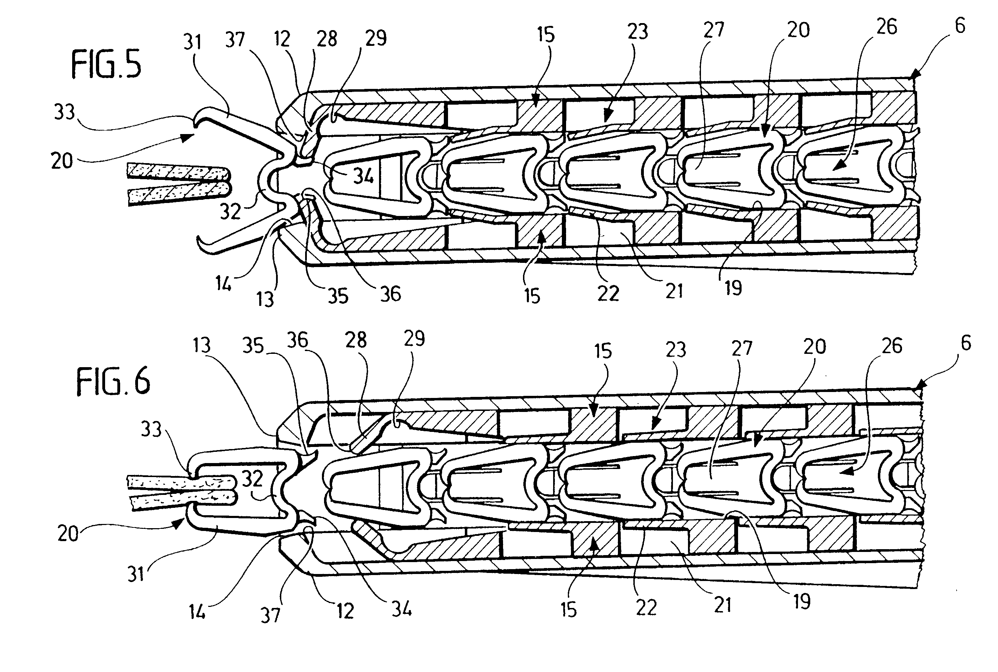

Durch die Bewegung der freien Kanten 36 der Wandabschnitte 28, die sich bei dieser Vorschubbewegung der Vorschubelemente 15 einander annähern, werden auch die Vorsprünge 34 der Clips 20 einander angenähert, und dies führt in der beschriebenen Weise zu einer Öffnung der Arme 31 entgegen der elastischen Schließkraft (Figur 5).

Sobald der Clip 20 mit den geöffneten Armen 31 die gewünschte Anlagestelle, beispielsweise die Schnittstelle der Kopfhaut, umgreift, kann der Clip 20 wieder geschlossen werden. Dazu genügt es, mittels des Betätitungsgriffes 3 die Vorschubelemente 15 wieder zurückzuziehen. Die Rückbewegung der Vorschubelemente 15 setzt sofort ein, wenn der Betätigungsgriff 3 wieder nach vorne verschwenkt wird, dabei wird die Rückbewegung des Vorschubelementes 15 durch die Federkraft des geöffneten und sich dann schließenden Clips 20 unterstützt.

Nach dem Schließen des Clips 20 und beim weiteren Zurückziehen der Vorschubelemente 15 treten die Wandabschnitte 28 mit ihrer freien Kante 36 wieder aus den Vertiefungen 25 der Vorsprünge 34 aus und geben den nunmehr in Anlageposition befindlichen Clip 20 frei (Figur 6). Beim weiteren Zurückschieben der Vorschubelemente 15 gleiten die Wandabschnitte 28 am nachfolgenden Clip entlang, der durch die an ihm anliegenden Federzungen 27 gegen ein Zurückschieben gesichert ist. Die freien Kanten 36 der Wandabschnitte 28 gelangen am Ende der Rückzugbewegung in die Vertiefungen 35 an den Vorsprüngen 34 dieses Clips 20, der dann in der beschriebenen Weise beim nächsten Vorschubtakt in der gewünschten Position angelegt werden kann.

Die Dimensionierungen sind so gewählt, daß die Mitnehmer 23 bei vollständig zurückgezogenem Vorschubelement 15 von den vor ihnen liegenden Clips 20 einen Abstand einhalten, während die Wandabschnitte 28 mit der freien Kante 36 bereits in der Vertiefung 35 des vordersten Clips 20 ruhen. Bei der Vorschubbewegung der Vorschubelemente 15 wird daher zunächst nur der vorderste Clip nach vorne geschoben und erst danach legen sich die Mitnehmer 23 an den übrigen Clips an und schieben diese nach vorn. Dadurch wird der Abstand zwischen dem vordersten Clip und dem nachfolgenden Clip vergrößert, so daß für das Eintauchen der die Öffnung der Clips bewirkenden Wandabschnitte 28 genügend Platz geschaffen wird.

Sobald das Magazin in der beschriebenen Weise geleert ist, kann es vom Handstück 1 abgenommen und durch ein neues, gefülltes Magazin ersetzt werden.

Es ist günstig, wenn zumindest die Vorschubelemente 15 einstückig aus einem elastischen, sterilisierbaren Kunststoff gefertigt sind, auch das Gehäuse 6 kann aus einem sterilisierbaren Kunststoff bestehen. Die Träger 26 mit den Federzungen 27 werden vorzugsweise als Metallstreifen ausgebildet.

## Patentansprüche

1. Magazin für ein chirurgisches Clipanlegegerät mit einem Aufnahmeraum (19) für hintereinander angeordnete und im Aufnahmeraum (19) zu dessen offenem vorderen Ende (12) hin verschiebbare Clips (20), die zwei über einen Steg (32) verbundene, gegen eine elastische Kraft in eine Öffnungsstellung auseinanderschwenkbare Arme (31) und diesen zugeordnete, über den Steg (32) nach hinten vorstehende Vorsprünge (34) aufweisen, und mit zwei einander gegenüberliegenden, zangenartig gegeneinander bewegbaren, an den Vorsprüngen (34) des jeweils vordersten Clips (20) anlegbaren Öffnungswerkzeugen (28), **dadurch gekennzeichnet, daß** die Öffnungswerkzeuge (28) scharnierartig mit einem im Magazin parallel zur Verschieberichtung der Clips (20) verschiebbaren Vorschubelement (15) verbunden sind und bei dessen in Richtung auf das offene, vordere Ende (12) des Aufnahmeraumes (19) gerichteter Vorschubbewegung an einer Umlenkfläche (37) des Magazins zur Anlage gelangen und dadurch quer zur Verschieberichtung zum jeweils gegenüberliegenden Öffnungswerkzeug (28) einschwenkbar sind.

2. Magazin nach Anspruch 1, **dadurch gekennzeichnet, daß** die Öffnungswerkzeuge (28) gegen eine elastische Rückstellkraft nach außen verschwenkbar sind, die geringer ist als die zum Öffnen der Clips (20) nötige Kraft.

3. Magazin nach einem der Ansprüche 1 oder 2, **dadurch gekennzeichnet, daß** die Öffnungswerkzeuge (28) über einen geschwächten Scharnierbereich (29) einstückig mit den Vorschubelementen (15) verbunden sind.

4. Magazin nach Anspruch 3, **dadurch gekennzeichnet, daß** die Öffnungswerkzeuge (28) und das Vorschubelement (15) aus einem elastischen Kunststoffmaterial bestehen.

5. Magazin nach einem der voranstehenden Ansprüche, **dadurch gekennzeichnet, daß** die Umlenkfläche (37) durch die sich zu beiden Seiten an das offene Ende (12) des Aufnahmeraumes (19) anschließende Stirnwand (13) des Magazins gebildet wird.

6. Magazin nach einem der voranstehenden Ansprüche, **dadurch gekennzeichnet, daß** das Vorschubelement (15) an die Clips (20) anlegbare Mitnehmer (23) zum schrittweisen Verschieben der Clips (20) aufweist.

7. Magazin nach Anspruch 6, **dadurch gekennzeichnet, daß** sich die Öffnungswerkzeuge (29) beim Vorschieben des Vorschubelementes (15) an den Vorsprüngen (34) des vordersten Clips (20) anlegen, bevor die Mitnehmer (23) des Vorschubelementes (15) sich an die folgenden Clips (20) anlegen.

8. Magazin nach Anspruch 6oder 7, **dadurch gekennzeichnet, daß** die Mitnehmer (23) beim Zurückziehen des Vorschubelementes (15) elastisch an den Clips (20) entlanggleiten.

9. Magazin nach Anspruch 8, **dadurch gekennzeichnet, daß** die Mitnehmer (23) als Federzungen (22) ausgebildet sind, die in Vorschubrichtung schräg in den Aufnahmeraum (19) hineinragen.

10. Magazin nach einem der voranstehenden Ansprüche, **dadurch gekennzeichnet, daß** die Vorschubelemente (15) als Halbschalen ausgebildet sind, die gemeinsam den kanalförmigen Aufnahmeraum (19) für die Clips (20) bilden.

11. Magazin nach Anspruch 10, **dadurch gekennzeichnet, daß** die Halbschalen in Vorschubrichtung relativ zueinander festgelegt sind.

12. Magazin nach einem der Ansprüche 10 oder 11, **dadurch gekennzeichnet, daß** die Halbschalen in einem länglichen Gehäuse (6) des Magazins in dessen Längsrichtung verschiebbar gelagert sind.

13. Magazin nach Anspruch 12, **dadurch gekennzeichnet, daß** die Halbschalen durch das Gehäuse (6) in gegenseitiger Anlage gehalten sind.

14. Magazin nach einem der voranstehenden Ansprüche, **dadurch gekennzeichnet, daß** in den Aufnahmeraum (19) aus diesem ausrückbare Rückhaltevorsprünge (27) für die Clips (20) hineinragen, die in Längsrichtung des Magazins festgelegt sind.

15. Magazin nach Anspruch 14, **dadurch gekennzeichnet, daß** die Rückhaltevorsprünge (27) federnd aus dem Aufnahmeraum (19) herausbewegbar sind.

16. Magazin nach Anspruch 15, **dadurch gekennzeichnet, daß** die Rückhaltevorsprünge (27) Federzungen sind, die in Vorschubrichtung schräg in den Aufnahmeraum (19) hineinragen.

17. Magazin nach einem der Ansprüche 14 bis 16, **dadurch gekennzeichnet, daß** die Rückhaltevorsprünge (27) an den Seitenwänden des Aufnahmeraumes (19) angeordnet sind.

18. Magazin nach Anspruch 17, **dadurch gekennzeichnet, daß** die Rückhaltevorsprünge (27) an Trägern (26) gehalten sind, die in den Aufnahmeraum (19) seitlich eingeschoben und die axial unverschieblich am Magazin festgelegt sind.

19. Magazin nach einem der voranstehenden Ansprüche, **dadurch gekennzeichnet, daß** die Vorschubelemente (15) an ihrer Außenseite in Längsrichtung aneinander anschließende Vertiefungen (21) aufweisen, in die ein in Verschieberichtung der Clips (20) vorund zurückschiebbarer Transportmitnehmer (25) eingreift.

20. Magazin nach Anspruch 19, **dadurch gekennzeichnet, daß** der Boden (22) der Vertiefung (21) an drei Seiten vom Vorschubelement (15) abgetrennt ist und den in den Aufnahmeraum (19) hineinragenden Mitnehmer (23) für die Clips (20) bildet.

## Claims

1. A magazine for a surgical clip applicator, comprising a holding space (19) for clips (20) which are arranged one behind the other and which are displaceable in the holding space (19) towards the open front end (12) thereof, the clips (20) having two arms (31) which are connected by a crosspiece (32) and are pivotable apart against a resilient force into an open position, and having projections (34) associated with the arms (31) and projecting rearwards beyond the crosspiece (32), and comprising two opposing opening tools (28) which are movable towards one another in the manner of forceps and which rest against the projections (34) of the respective frontmost clip (20), **characterised in that** the opening tools (28) are connected in a hinged manner to a feed member (15) which is displaceable in the magazine parallel to the displacement direction of the clips (20) and, during the feed movement of the feed member (15) towards the open front end (12) of the holding space (19), come to rest against a deflection surface (37) of the magazine and are thereby pivotable inwards transversely to the displacement direction towards the respective opposing opening tool (28).

2. A magazine according to claim 1, **characterised in that** the opening tools (28) are pivotable outwards against a resilient restoring force which is lower than the force necessary for opening the clips (20).

3. A magazine according to either one of claims 1 and 2, **characterised in that** the opening tools (28) are integrally connected to the feed members (15) via a weakened hinge region (29).

4. A magazine according to claim 3, **characterised in that** the opening tools (28) and the feed member (15) are made of a resilient plastics material.

5. A magazine according to any one of the preceding claims, **characterised in that** the deflection surface (37) is formed by the end wall (13) of the magazine, the end wall (13) being adjacent to the open end (12) of the holding space (19) on both sides.

6. A magazine according to any one of the preceding claims, **characterised in that** the feed member (15) has carriers (23) which are placeable against the clips (20) and are provided for the progressive displacement thereof.

7. A magazine according to claim 6, **characterised in that**, during the feed movement of the feed member (15), the opening tools (28) rest against the projections (34) of the frontmost clip (20) before the carriers (23) of the feed member (15) come to rest against the following clips (20).

8. A magazine according to claim 6 or 7, **characterised in that** the carriers (23) slide resiliently along the clips (20) when the feed member (15) is retracted.

9. A magazine according to claim 8, **characterised in that** the carriers (23) are formed as resilient tongues (22) projecting obliquely into the holding space (19) in the feed direction.

10. A magazine according to any one of the preceding claims, **characterised in that** the feed members (15) are formed as half-shells which together form the channel-type holding space (19) for the clips (20).

11. A magazine according to claim 10, **characterised in that** the half-shells are fixed relative to one another in the feed direction.

12. A magazine according to either one of claims 10 and 11, **characterised in that** the half-shells are mounted in an elongate housing (6) of the magazine so as to be displaceable in the longitudinal direction thereof.

13. A magazine according to claim 12, **characterised in that** the half-shells are held in mutual contact by the housing (6).

14. A magazine according to any one of the preceding claims, **characterised in that** retaining projections (27) for the clips (20) project into the holding space (19) and are disengageable therefrom and are fixed in the longitudinal direction of the magazine.

15. A magazine according to claim 14, **characterised in that** the retaining projections (27) are resiliently movable out of the holding space (19).

16. A magazine according to claim 15, **characterised in that** the retaining projections (27) are resilient tongues projecting obliquely into the holding space (19) in the feed direction.

17. A magazine according to any one of claims 14 to 16, **characterised in that** the retaining projections (27) are arranged on the side walls of the holding space (19).

18. A magazine according to claim 17, **characterised in that** the retaining projections (27) are held on supports (26) which are inserted laterally into the holding space (19) and are axially non-displaceably fixed to the magazine.

19. A magazine according to any one of the preceding claims, **characterised in that** the feed members (15) have, on their outer surface, recesses (21) which adjoin one another in the longitudinal direction and in which engages a driver (25) which is movable back and forth in the displacement direction of the clips (20).

20. A magazine according to claim 19, **characterised in that** the base (22) of the recess (21) is separated from the feed member (15) on three sides and forms the carrier (23) for the clips (20), the carrier (23) projecting into the holding space (19).

## Revendications

1. Magasin pour un appareil de pose d'agrafes chirurgicales avec un espace de logement (19) pour des agrafes (20) disposées les unes derrière les autres et mobiles dans l'espace de logement (19) vers l'extrémité avant ouverte (12) de celui-ci, qui présentent deux bras (31) reliés par l'intermédiaire d'une barrette (32), pouvant s'écarter l'un de l'autre contre une force élastique pour prendre une position d'ouverture, et des saillies (34) associées aux-dits bras, dépassant vers l'arrière au-delà de la barrette (32), et avec deux outils d'ouverture (28) situés de manière opposée l'un à l'autre, mobiles l'un vers l'autre à la manière d'une pince, pouvant être posés contre les saillies (34) de l'agrafe (20) située le plus en avant, **caractérisé en ce que** les outils d'ouverture (28) sont reliés à la manière d'une charnière avec un élément d'avance (15) mobile dans le magasin parallèlement à la direction de déplacement des agrafes (20) et viennent reposer contre une surface de déviation (37) du magasin lors du mouvement d'avance de l'élément d'avance en direction de l'extrémité avant ouverte (12) de l'espace de logement (19) et de ce fait peuvent pivoter vers l'intérieur, transversalement à la direction de déplacement, chacun vers l'outil d'ouverture (28) opposé.

2. Magasin selon la revendication 1, **caractérisé en ce que** les outils d'ouverture (28) peuvent pivoter vers l'extérieur contre une force de rappel élastique qui est plus faible que la force nécessaire pour ouvrir les agrafes (20).

3. Magasin selon l'une des revendications 1 ou 2, **caractérisé en ce que** les outils d'ouverture (28) sont reliés d'une seule pièce avec les éléments d'avance (15) par l'intermédiaire d'une zone charnière affaiblie (29).

4. Magasin selon la revendication 3, **caractérisé en ce que** les outils d'ouverture (28) et l'élément d'avance (15) sont réalisés dans une matière plastique élastique.

5. Magasin selon l'une quelconque des revendications précédentes, **caractérisé en ce que** la surface de déviation (37) est formée par la paroi frontale (13) du magasin faisant suite des deux côtés à l'extrémité ouverte (12) de l'espace de logement (19).

6. Magasin selon l'une quelconque des revendications précédentes, **caractérisé en ce que** l'élément d'avance (15) présente des doigts d'entraînement (23) pouvant être posés contre les agrafes (20) pour déplacer les agrafes (20) progressivement.

7. Magasin selon la revendication 6, **caractérisé en ce que** les outils d'ouverture (29) se posent contre les saillies (34) de l'agrafe située le plus en avant (20) lors de l'avance de l'élément d'avance (15), avant que les doigts d'entraînement (23) de l'élément d'avance (15) se posent contre les agrafes suivantes (20).

8. Magasin selon la revendication 6 ou 7, **caractérisé en ce que** les doigts d'entraînement (23) glissent élastiquement le long des agrafes lors du recul de l'élément d'avance (15).

9. Magasin selon la revendication 8, **caractérisé en ce que** les doigts d'entraînement (23) sont formés comme des languettes élastiques (22) qui pénètrent à l'oblique dans l'espace de logement (19) dans la direction d'avance.

10. Magasin selon l'une quelconque des revendications précédentes, **caractérisé en ce que** les éléments d'avance (15) sont formés comme des demi-coques qui forment ensemble l'espace de logement (19) en forme de canal pour les agrafes (20).

11. Magasin selon la revendication 10, **caractérisé en ce que** les demi-coques sont fixées l'une par rapport à l'autre dans la direction d'avance.

12. Magasin selon l'une des revendications 10 ou 11, **caractérisé en ce que** les demi-coques sont montées dans un boîtier allongé (6) du magasin, de manière mobile dans la direction longitudinale de celui-ci.

13. Magasin selon la revendication 12, **caractérisé en ce que** les demi-coques sont maintenues en appui mutuel par le boîtier (6).

14. Magasin selon l'une quelconque des revendications précédentes, **caractérisé en ce que** des saillies de retenues (27) pour les agrafes (20) qui sont fixées dans le sens longitudinal du magasin, pénètrent dans l'espace de logement (19) et peuvent se dégager de celui-ci.

15. Magasin selon la revendication 14, **caractérisé en ce que** les saillies de retenue (27) peuvent être sorties de l'espace de logement (19) de manière élastique.

16. Magasin selon la revendication 15, **caractérisé en ce que** les saillies de retenue (27) sont des languettes élastiques qui pénètrent à l'oblique dans l'espace de logement (19) dans la direction d'avance.

17. Magasin selon l'une des revendications 14 à 16, **caractérisé en ce que** les saillies de retenue (27) sont disposées sur les parois latérales de l'espace de logement (19).

18. Magasin selon la revendication 17, **caractérisé en ce que** les saillies de retenue (27) sont tenues sur des supports (26) qui sont introduits latéralement dans l'espace de logement (19) et sont fixés au magasin de manière fixe dans le sens axial.

19. Magasin selon l'une quelconque des revendications précédentes, **caractérisé en ce que** les éléments d'avance (15) présentent sur leur face extérieure des cavités (21) adjacentes les unes aux autres dans la direction longitudinale, dans lesquelles s'insère un doigt d'entraînement de transport (25) pouvant être avancé et reculé dans la direction de déplacement des agrafes (20).

20. Magasin selon la revendication 19, **caractérisé en ce que** le fond (22) de la cavité (21) est séparé de l'élément d'avance (15) sur trois côtés et forme le doigt d'entraînement (23) pénétrant dans l'espace de logement (19) pour les agrafes (20).
